# EUROPÄISCHE PATENTANMELDUNG

(11) **EP 1 752 137 A2**
(43) Veröffentlichungstag der Anmeldung: **14.02.2007**
(21) Anmeldenummer: 06013550.6
(22) Anmeldetag: 30.06.2006
(51) Int. Cl.: A61K 8/92, A61K 8/60, A61Q 19/00, A61K 9/06

(54) **Hydrophile bzw. ambiphile Grundlagen und Zubereitungen**

(30) Priorität: 02.08.2005 DE 10536160
(71) Anmelder: Merz Pharma GmbH & Co.KGaA, 60318 Frankfurt (DE)
(72) Erfinder: Beutler, Rolf D., Dr., 64739 Höchst (DE); Nürnberg, Eberhard, Prof. Dr., 91080 Uttenreuth (DE); Sawiec, Joanna, Mag. Pharm., 91052 Erlangen (DE); Nürnber, Wolf, Dr. med. P.D., 18225 Kühlungsborn (DE)
(74) Vertreter: Müller, Claudia

(57) **Zusammenfassung**

Die vorliegende Erfindung betrifft neue Grundlagen auf Naturstoff- Basis, insbesondere Creme- oder Lotion- Zubereitungen, welche unabhängig von der gewünschten Indikation und Anwendung stabile Emulsionssysteme unter Verzicht auf (synthetische) herkömmliche Lipide sowie herkömmliche Emulgatorgemische darstellen. Sie umfassen insbesondere natürliche Grundstoffe, wie sie z. B. in der Lebensmittelverarbeitung zum Einsatz kommen und können Tensidassoziate und anisotrope Strukturen in Form von in Form von anisotropen Molekülassoziaten im Mikrometer und/oder Nanometerbereich, wie z.B. Nanosomen (Nanobionen) ausbilden. Sie können für kosmetische, pflegende, balneologische Anwendungen, vor allem auch für dermatologische, medizinische Anwendungen, auf insbesondere menschlicher Haut / Schleimhaut eingesetzt werden.

## Beschreibung

### Gegenstand der Erfindung

Die vorliegende Erfindung betrifft neue Grundlagen auf Naturstoff - Basis, insbesondere Creme- oder Lotion- Zubereitungen, welche unabhängig von der gewünschten Indikation / Anwendung stabile Emulsionssysteme unter Verzicht auf herkömmliche ((partial-)synthetische) Lipide sowie herkömmliche Emulgatorgemische darstellen. Sie umfassen natürliche Grundstoffe, wie sie z. B. in der Lebensmittelverarbeitung zum Einsatz kommen und können Tensidassoziate und anisotrope Strukturen in Form von anisotropen Molekülassoziaten im Mikrometer und/oder Nanometerbereich, wie z.B. Nanosomen (Nanobionen) ausbilden. Sie können für kosmetische, pflegende oder balneologische Anwendungen, vor allem auch für dermatologische, medizinische Anwendungen, auf insbesondere menschlicher Haut / Schleimhaut eingesetzt werden.

### Hintergrund der Erfindung

Cremes oder Lotionen, welche auf menschliche Haut aufgetragen werden sollen, basieren im wesentlichen auf einer Grundlage aus einer Fettphase, einer Wasserphase, ggf. unter Zusatz weiterer Stoffe wie ein- oder mehrwertige Alkohole, z. B. Glycerol, hochmolekulare Substanzen, sowie weiterhin einem Gemisch aus Emulgatoren, die in der Regel aus lipophilen W/O- und hydrophilen O/W- Tensiden bestehen. Während für die Herstellung von Fettcremes (lipophile W/O- Cremes), die durch eine kohärente Lipidphase gekennzeichnet sind, zur Inkorporierung einer inkohärenten Wasserphase nur der Zusatz von W/O- Emulgatoren erforderlich ist, benötigt man üblicherweise zur Herstellung von O/W- Produkten Mischemulgatoren aus mehr oder weniger wasserlöslichen O/W- und wasserunlöslichen W/O- bzw. wasserdispergierbaren Emulgatoren (Mischemulgatoren). Nur in wenigen Ausnahmefällen ist der Zusatz eines einzelnen Emulgators, der die Eigenschaften von O/W- und W/O Tensiden in sich vereinigt, zur Gewinnung von hydrophilen Cremes möglich, siehe DE 41 21 945, betreffend den Einsatz nichtionischer polyoxyethylierter Tenside. Es können auch W/O Emulgatoren zur Stabilisierung hinzugesetzt werden. Insbesondere werden allgemein anionische Tenside wie Natriumlaurylsulfat, nichtionische Tenside wie Polysorbate, ethoxylierte Produkte des Macrogol-Typs und Cetylstearylalkohole, Wollwachsalkohole als W/O- bzw. Co- Emulgatoren eingesetzt, wobei keine Beschränkung auf diese Substanzen besteht. Herkömmliche, gängige Fettphasen (synthetische, partialsynthetische Lipide) sind solche auf Paraffin- und Vaselinbasis. Allerdings sind gerade übliche Co- Emulgatoren als Auslöser von Kontaktallergien bekannt, vgl. O. Braun- Falko, M. Gloor, H.C. Korting, "Nutzen und Risiko von Kosmetika", 2000 S. 57 und 49. Wenngleich auch der Aufbau derartiger Emulsionssysteme im Grunde relativ einfach ist, so ist angesichts der Vielzahl vorhandener und potentiell geeigneter Lipide und Emulgatoren bzw. Tenside eine extrem hohe Anzahl von Grundlagen für therapeutisch und kosmetisch topisch anwendbare Systeme im Handel. Der Anwender muss somit je nach beabsichtigtem Zweck die entsprechend spezifisch formulierten Produkte zum Beispiel mit unterschiedlichen Penetrationsvermögen oder Pflegeeigenschaften auswählen. Anwendungsorientierte Eigenschaften werden dabei vielfach durch den Zusatz bestimmter Hilfsstoffe erzielt. Dementsprechend hoch ist auch die Anzahl derartiger Hilfsstoffe in den Produkten. Da ferner der therapeutische und auch kosmetologisch erwünschte Erfolg in starkem Maße von der Grundlage abhängt, werden für ein und dieselbe Indikation verschiedenartige Grundlagen zur Verfügung gestellt. In jedem Fall kann es durch die Vielzahl von Hilfsstoffen aber auch insbesondere durch die Emulgatorsysteme selbst zu unerwünschten Wechselwirkungen mit der Haut kommen.

### Aufgabenstellung

Der vorliegenden Erfindung liegt daher die Aufgabe zugrunde, eine hydrophile bzw. ambiphile Emulsionsgrundlage bereitzustellen, welche sich durch eine geringst mögliche Zahl an Hilfsstoffen und damit ein minimiertes Risiko von Irritationen und Sensibilisierungsreaktionen und insbesondere durch ein anwendungsorientiertes geeignetes Emulsionssystem auszeichnet. Insbesondere sollen dabei biologische Grundlagenkomponenten auf Basis von genuinen Naturstoffen und solchen Substanzen, die beim physiologischen Abbau (Biotransformation) in pharmakologisch-physiologisch indifferente Verbindungen überführt werden, zum Einsatz kommen. Unter ambiphilen Emulsionssystemen werden dabei solche Zubereitungen verstanden, die sowohl mit Wasser bzw. wässrigen Lösungen als auch mit lipophilen Substanzen wie fetten Ölen, prinzipiell mischbar sind. Hydrophile Systeme sind demnach solche Zubereitungen, die nur mit Wasser bzw. wässrigen Lösungen mischbar sind.

### Lösung der Aufgabe

Diese Aufgabe wird erfindungsgemäß dadurch gelöst, dass man flüssige oder streichfähige d.h. salbenartige, stabile Grundlagen auf Basis natürlicher, insbesondere essbarer Ausgangmaterialien bereitet und dabei im wesentlichen auf bisher eingesetzte synthetische oder partialsynthetische Substanzen ((partial-)synthetische Emulgator-Substanzen bzw. Lipid- Substanzen) verzichten kann. Die Grundlagen umfassen eine Wasserphase und ein oder mehrere Emulsionsstabilisatoren, ausgewählt aus Kohlenhydratverbindungen wie Zuckerestern und Zuckerethern. Hierdurch wird erfindungsgemäß bereits eine flüssig-viskose oder salbenartige streichfähige Form erhalten, wobei ein System entsteht, in welchem die Fett(ÖI)phase im wesentlichen aus natürlichen Lipiden besteht. Überraschenderweise kann auf diese Weise ein stabiles System auch ohne den Zusatz synthetischer / partialsysnthetischer Co- Emulgator- Substanzen wie Cetyl- / Stearylalkohol, oder auch Partialestern des Glycerols und anderer mehrwertiger Alkohole mit höheren Fettsäuren erhalten werden. Die beschriebenen Grundlagen sind insofern vorzugsweise im wesentlichen frei von derartigen Co-Emulgatoren. Die beschriebenen Grundlagen können dann vor allem auch durch Inkorporierung von geeigneten Wirkstoffen als Cremes und Lotionen mit einer gewünschten Indikation verwendet werden. Unerwarteter Weise können mit derartigen Systemen in geeigneten Verhältnissen von Fettphase, Wasserphase, Zuckerverbindung auch anisotrope Assoziate bzw. Strukturen in Form von Biosomen, d.h. Mikrosomen (Mikrobionen) und/oder Nanosomen (Nanobionen) erhalten werden. Als Biosome werden normalerweise optisch anisotrope Assoziate, z. B. mit Cholesterol, gegebenenfalls mit Zusatz weiterer Substanzen, wie Phospholipiden und Wirkstoffen bezeichnet. Sie liegen im Partikelgrößenbereich vom Nanometer und Mikrometer - bis ca. 50 µm. Gemäß vorliegender Erfindung können derartige Strukturen, welche analog liposomaler Strukturen einen besseren Stofftransport bewirken können, erhalten werden, allerdings mit dem Unterschied, dass hier nicht zwingend zusätzliche spezielle Substanzen wie z.B. Phospholipide und Sterole von Typ des Cholesterols erforderlich sind. Die erhaltenen Zubereitungen sind aufgrund der speziellen Zusammensetzung hypoallergen, d.h. mit einem sehr geringen Sensibilisierungspotential belastet und daher vorzüglich verträglich. Sie eignen sich daher sowohl zur Pflege / Behandlung im kosmetischen Bereich, wie beispielsweise bei trockener, alternder, reifer, gereizter, gestresster Haut, zur Reinigung z. B. als Badezusatz oder auch als Sonnenschutzmittel. Sie können auch im dermatologisch/ medizinischen Bereich wie z. B. als Antimykotikum, Antiallergikum eingesetzt werden.

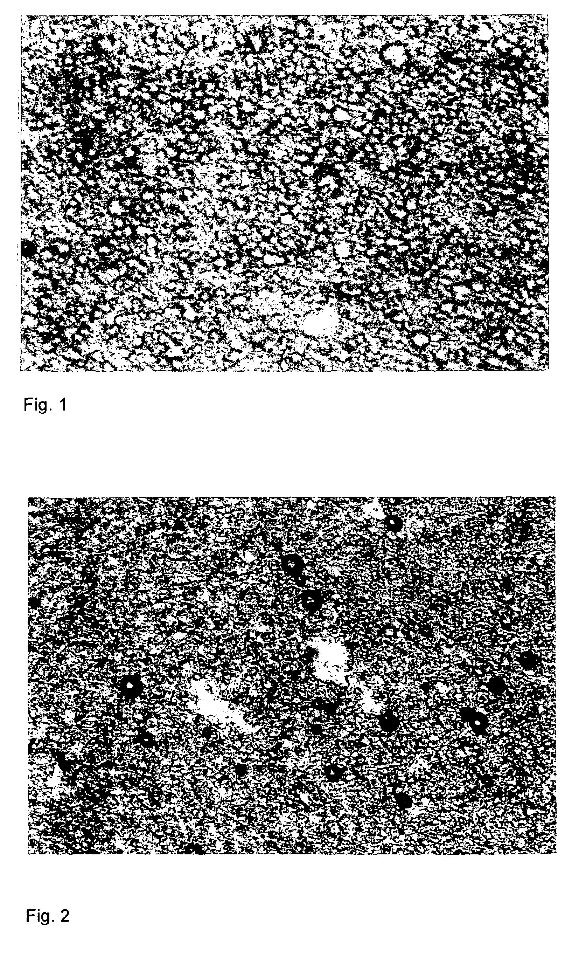

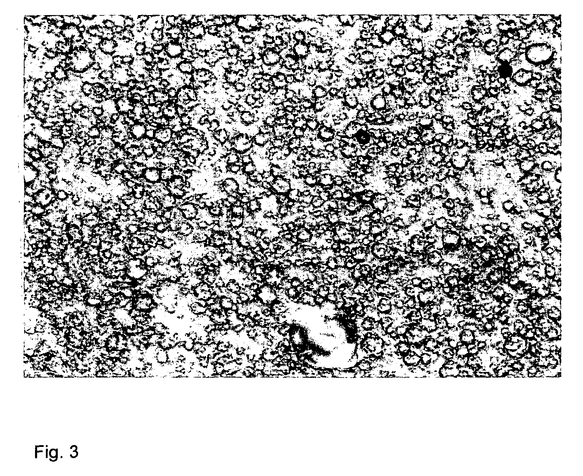

### Beschreibung der Abbildungen Fig.1-3

In den Fig. 1 bis 3 sind Polarisationsmikroskopische Aufnahmen von erfindungsgemäßen Grundlagen gemäß den Beispielen 9-11 gezeigt, welche die anisotropen Assoziatstrukturen bzw. Biosomen als helle Punkte bzw. Zonen (Strukturverdichtung) abbilden.

### Nähere Erläuterung der Erfindung

Die Erfindung betrifft insofern Grundlagen mit unterschiedlich ausgeprägter Hydrophilie bzw. Lipophilie für pharmazeutische, kosmetische und pflegende Zubereitungen in Formen von Cremes und Lotionen, auf natürlicher Basis, enthaltend
a) 1 bis 50 % eines oder mehrerer Lipide (Fettphase), ausgewählt aus nativen flüssigen, halbfesten oder festen Lipiden, vorzugsweise Pflanzenölen bzw. Pflanzenwachsen;
b) 0,1 bis 30 % eines oder mehrerer Emulsionsstabilisatoren, ausgewählt aus
   bi) Zuckerestern (Zuckerester, substituierte Zuckerester, insbesondere nieder-Alkylsubstituierte Zuckerester wie vor allem Methylglucose) von C₁₂-C₁₈ gesättigten, ungesättigten, partial gesättigten Fettsäuren oder Mischungen hiervon;
   bii) Zuckerethern von C₈-C₂₀ gesättigten, ungesättigten, partial gesättigten Fettalkoholen oder Mischungen hiervon; oder Mischungen aus bi), bii);
c) 10 bis 95 Gew.% Wasser;
d) 0 bis 55 Gew.% Zusatzstoffe.

Hierbei sind unter "Emulsionsstabilisatoren" solche Substanzen zu verstehen, die geeignet sind, homogene Emulsionen zu gewinnen, zu begünstigen und zu stabilisieren. Die Menge an Emulsionsstabilisator(en) beträgt insgesamt 0,1 bis 30 Gew.%, bevorzugt 1 -20 Gew.%, vor allem 0,1 bis 15 Gew.%, insbesondere 0,5 bis 15, und vor allem 1 bis 12 Gew. %. In einer weiteren Ausführungsform beträgt die Menge an Emulsionsstabilisator(en) insbesondere 1 bis 15, vor allem 5 bis 13 Gew.%. Zur Charakterisierung der Kohlenhydrat-Emulsionsstabilisatoren kann die durch DETA-Analyse (Dielektrische Thermoanalyse) ermittelte Permittivität (ε') herangezogen werden. Die ε'-Werte liegen für die Reinsubstanzen je nach Polarität insbesondere zwischen 1 und 12, ggf. auch höher.
Bevorzugt sind 1 bis 50 Gew.%, insbesondere 5 bis 40 Gew.%, eines oder mehrerer Lipide, und 0,1 bis 30 Gew.% , insbesondere 1-25 Gew.%, vor allem auch 1 bis 20 Gew.%, Emulsionsstabilisator(en) enthalten. Ganz besonders bevorzugt sind 1 bis 25 Gew.% Emulsionsstabilisatoren, in Kombination mit 5-40 Gew.% Lipid, vorhanden oder auch Zusammensetzungen vor allem mit 2 bis 20 Gew. % Emulsionsstabilisatoren, insbesondere in Kombination mit 1 bis 20 Gew. % Zusatzstoffen.
Die Erfindung betrifft insofern insbesondere kosmetische, pflegende, balneologische und/ oder dermatologische Zubereitungen auf Basis der oben beschriebenen Grundlagen. Bevorzugt sind solche, wobei z. B. 45 bis 100, insbesondere 45 bis 99 Gew. %, vor allem 80 bis 99,9 Gew.% an a+b+c) zusammen mit 0 bis 55, insbesondere 0,1 bis 55, vor allem 1 bis 55 bzw. 0,1 bis 20 Gew. % Zusatzstoffen vorliegen, welche vor allem geeignete Wirkstoffe sein können.

Die Grundlage umfasst bevorzugt 2 bis 50 Gew. %, insbesondere 5 bis 40 Gew.%, eines oder mehrerer Lipide.
Besonders bevorzugt sind Emulsionsstabilisatoren, deren Zuckerteil ausgewählt ist aus Mono-, Di- oder Oligosachcariden wie -Glucose, -Methylglucose, -Fructose, -Maltose, -Saccharose, - Lactose, oder Polymeren Sacchariden mit geringern DP (Durchschnittspolymerisationsgrad) von vorzugsweise etwa 1-2.
Besonders bevorzugte Emulsionsstabilisatoren sind hier Glucose-, Methylglucose- oder Saccharose- C₁₂-C₁₈-Säureester; C₈-C₂₀-Glukoseether oder Mischungen hiervon.
Zusatzstoffe, welche bevorzugt in einer Menge von 0 bis 55 Gew.%, vor allem 1-25 Gew.%, insbesondere 1 bis 10 Gew.% in den kosmetisch / dermatologischen Zubereitungen vorliegen, werden insbesondere ausgewählt aus einem oder mehreren Wirkstoffen wie insbesondere pflegenden, kosmetischen und / oder therapeutisch wirksamen Stoffen, ferner auch aus der Gruppe, umfassend Konsistenzregler, Konservierungsstoffe, Farbstoffe, Parfümstoffe, Schaumregler oder Mischungen hiervon oder Mischungen hiervon und / oder mit einem oder mehreren Wirkstoffen. Geeignete Wirkstoffe sind beispielsweise ätherische Öle, Pflanzenextrakte, und / oder fossile Stoffe wie Torfprodukte, UV Filter, Antibiotika, Antimykotika, Virostatika, Antiseptika, haarwuchsfördernde Substanzen, entzündungshemmende Stoffe, Hormone, Antischuppenmittel, zellschützende und antioxidative Substanzen, Mineralien, Vitamine, oder Mischungen hiervon, Pflegestoffe, Feuchthalter, Rückfetter, ein oder mehrere Lipidtransferproteine, insbesondere ausgewählt aus pflanzlichen Proteinen, Milchproteinen und Mischungen hiervon, ein oder mehreren Lecithine oder Lecithinanaloga, Cholesterine, Phospholipide, Sphingolipide, Ganglioside, Cerebroside, Ceramide, insbesondere Kombinationen von Lipidtransferprotein(en) und Lecithinen/Phospholipiden etc., letztere auch vor allem in Kombination mit einem oder mehreren fett- oder wasserlöslichen Vitaminen, oder Mischungen genannter Wirkstoffe.
In einer weiteren geeigneten Ausführungsform können als Wirkstoff vor allem auch Torfprodukte oder analoge Huminsäurequellen, insbesondere mit geeigneten Zusatzstoffen wie Schaumreglern eingesetzt werden.

### Nähere Beschreibung der Inhaltsstoffe

### 1. Lipidphase

Als Lipidphase eignen sich insbesondere vegetabilische Pflanzenfette und Pflanzenöle wie sie in der Nahrungsmittelindustrie eingesetzt werden, nämlich: Mandelöl, Sojaöl, Kokosnussöl, Jojobaöl, Avocadoöl, Erdnussöl, Traubenkernöl, Sonnenblumenöl, Borretschöl, Sesamöl, Olivenöl, Aprikosenkernöl, Maiskeimöl, Weizenkeimöl, Walnussöl, Palmöl, Macadamianussöl, Palmkernöl, Haselnussöl, Hagebuttenkernöl, Baumwollsamenöl.
Gegebenenfalls kann es vorteilhaft sein, auch zusätzlich aus natürlichen Substanzen gewonnene Fettsäureester wie mittelkettige (z. B. C₈₋₁₂) Triglyceride, mittelkettige (C₈₋₁₂) mittelkettige Propylenglyokolester einzusetzen.

### 2. Emulsionsstabilisatoren

Als Emulsionsstabilisatoren werden Zuckerester und/oder Zuckerether der folgenden Art gewählt:
Zuckerester von gesättigten, ungesättigten, partialgesättigten C₁₂-C₁₈-Fettsäuren. Als Kohlenhydrate kommen dabei Mono-, Di- und/oder Oliago-Saccharide oder auch substituierte, insbesondere nieder- Alkyl-, vor allem CH₃substituierte Derivate hiervon infrage. Insbesondere bevorzugt sind Glucose, Methylglucose, Fruktose, Saccharose. Besonders bevorzugt sind Glukose, Methylglucose und Saccharose. Als Fettsäuren sind vor allem organische aliphatische Carbonsäuren geeignet. Hierzu gehören insbesondere Caprylsäure, Caprinsäure, Laurinsäure, Myristinsäure, Palmitinsäure, Stearinsäure, Ölsäure und auch Mischungen dieser. Besonderes bevorzugt sind hier C₁₂-C₁₈-Säuren, welche mit Glucose, Methylglucose oder Saccharose verestert sind. Saccharoseester sind vorwiegend Sucrose-stearat, -palmitat, -laurat oder -oleat. Ganz besonderes bevorzugt sind C₁₆/C₁₈ Zuckerester, insbesondere in einer Menge von 0,1 bis 10 Gew.%, vor allem Saccharosestearat/palmitat, z.B. Crodesta^{®} F160, F110 oder F20.
Eine weitere Gruppe geeigneter Zucker- Emulsionsstabilisatoren stellen die Zuckerether von gesättigten, ungesättigten, partial gesättigten C₈-C₂₀- Fettalkoholen dar. Hierbei handelt es sich um Glucoside aus den vorgenannten Zuckern und ein- oder mehrwertigen Alkoholen mit C₈- bis C₂₀ Kettenlänge. Ganz besonders geeignet sind hier Glukoseether mit einer bevorzugten Kettenlänge im Alkylteil von ca. C₈-C₂₀, vor allem C₁₀C₁₄-Zuckerether, und vor allem die hierzu unter dem Namen Plantacare^{®} beschriebenen Zuckerether- Produkte, insbesondere Decylglucosid (Plantacare^{®} 2000 UP).
Diese werden bevorzugt in einer Menge von 0,3 bis 10 Gew.% eingesetzt. Es kann auch vorteilhaft sein, wenn 20-95%, insbesondere 20- 70% Wasser (und ggf. Zusatzstoffe wie angegeben) vorhanden sind.

### 3. Zusatzstoffe

Durch Kombination mit geeigneten Zusatzstoffen, insbesondere Wirkstoffen, erhält man pflegende, kosmetische, balneologisch oder dermatologisch einsetzbare Zubereitungen. Je nach Wirkstoff kann somit mit gleicher Grundlage eine unterschiedliche Anwendung erfolgen. So ist der Zusatz anwendungsorientierter Stoffe wie z. B. kosmetischer- oder hautpflegend wirksamer Komponenten, spreitungs- oder auch schaumbildender bzw. verstärkender Komponenten auf Basis der beschriebenen erfindungsgemäßen Grundlagen möglich. Geeignete Zusatzstoffe werden nachfolgend erläutert:
Als Zusatzstoffe kommen insbesondere infrage:
1) Konsistenzregler
   wie Celluloseether, z.B. Methylcellulose, Hydroxyethylcellulose, Hydroxypropylcellulose, Stärke und Stärkeabkömmlinge, z.B. lösliche oder quellbare Stärke, oxidierte Stärke; Stärkephosphat, Hydroxypropylstärke, Kolloide wie Guar Gum, Johannisbrotkernmehl, Guarkernmehl, Xanthan, Carragen, Gummi arabicum, Amylopektin, Amylose, Tragant, Alginat; pflegende Konsistenzregler und Wachse wie z.B. Shea butter (Butyrospermum parkii), Kakaobutter, Bienenwachs, Carnaubawachs; pH-Wert Regulatoren;
2) Parfümstoffe
   Geeignet sind hier: synthetische, naturidentische oder natürliche Parfüm- und Aromastoffe oder ätherische Öle z.B. Rosenöl, Geranienöl, Melissenöl, die sich beispielsweise aus Linalool, Geraniol, Citral, Citronellal zusammensetzen können.
3) Farbstoffe:
   Hier kommen die dem auf dem angesprochenen Gebiet bekannten Substanzen, insbesondere auch natürlichen Ursprungs infrage;
4) Konservierungsstoffe
   Geeignet sind beispielsweise Alkohole wie Ethanol, Benzoesäure bzw. deren Salze, Propionsäure bzw. deren Salze, Salicylsäure bzw. deren Salze, Sorbinsäure bzw. deren Salze, 1,3-Butandiol, Benzylalkohol, Benzylbenzoat, Phenylethanol, 4-Hydroxybenzoesäure-methyl-, -ethyl-, -propyl-, -butylester; Terpene aus ätherischen Ölen, insbesondere solche mit Alkohol- und / oder Aldehydfunktionen wie z. B. Citronelol, Citral, Citronelal; Linalool, Geraniol; Antioxidantien, wie Ascorbinsäure, α-Tocopherol, BHA, BHT, EDTA.
5) Schaumregler wie Betaine u.a. amphotere und / oder anionische Tenside, Macrogolabkömmlinge;
6) Wirkstoffe
   Diese sind insbesondere ausgewählt aus:
   Feuchthaltern wie Glycerol, Propylenglykol, Sorbitol, Harnstoff; Pflegestoffen wie Rückfettende Substanzen (z.B. Cetiol HE), Panthenol (Pantothenylalkohol), Allantoin, Bisabolol, Lecithin (Sojalecithin) sowie Ceramide und Sterole (z.B. Cholesterol) oder hautschützende Substanzen wie z.B. Polysiloxane;
   Vitaminen und deren Abkömmlingen, wie beispielsweise Vitamin A, E, C bzw. geeignete Derivate hiervon wie Ester z.B. Palmitat, Acetat oder Phosphat, Vitamin B₆ (Pyridoxin), Vitamin PP (Nicotinsäureamid);
   Weiterhin geeignete Wirkstoffe sind:
   Ätherische Öle und Extrakte aus Pflanzen wie beispielsweise solche aus Kamille, Calendula, Bambus, Johanniskraut, Amika, Zaubernuss, Ylang Ylang, Kiefernnadel, Zypresse, Thymian, Ginkgo, Melone, Minze, Limetten, Orangen, Grapefruit, Mandarine, Wacholder, Baldrian, Zitronenmelisse, Eukalyptus, Thymian, Palmarosa, Rosmarin, Lavendel, Rosenholz, Lemongras, Fichtennadel, Kiefernnadel, Ingwer-, Johannisbeer-, Lindenblüten-, Ringelblumen-, Magnolien-, Algen-, Aloe Vera-, Ananas-, Guave-, Echinacea-, Zimt-, Efeublätterxtrakt oder Mischungen hiervon; Extrakte aus Torf, Steinkohle, Braunkohle für Moorbäder, Natrium- und Ammoniumbitumino-sulfonate; Terpene und Terpenoide wie Campher, Menthol, Cineol oder andere aus den oben genannten Pflanzen erhältliche Terpene oder Mischungen hiervon;
   Wirkstoffe wie UV-Filter wie UVB, UVA und Breitbandfilter wie z.B. Ethylhexyl Methoxycinnamat, p-Methoxycinnamat, 4-Methylbenzyliden Campher, Paraaminobenzoesäureester wie N, N-Dimethyl-4-aminobenzoesäure-2-ethylhexylester Homomenthylsacilylat, Octyl Salicylate Octocrylene, Phenylbenzimidazolesulfonic Acid, Benzylidene Malonate Polysiloxane, Benzophenone-3; Methyl Anthranilate, Butyl Methoxydibenzoylmethane, Methylene Bis-Benzotriazolyl, Tetramethylbutylphenol, Bis-Ethylhexyloxyphenol Methoxyphenyltriazin, Disodium Phenyl Dibenzimidazole Tetrasulfonate, 2-(4-Diethylamino-2-hydroxybenzoyl) Benzoic Acid Hexylester, anorganische UV-Filter wie Zinkoxid und Titandioxid, insbesondere mikronisiert und/oder beschichtet;
   Für dermatologische Zwecke eignen sich vor allem Wirkstoffe wie Antimykotika, z.B. Clotrimazol, Ciclopiroxolamin oder Ketoconazol, Antiseptika, Antibiotika, z.B. Gentamicin, Hormone, hormonwirksame Substanzen bzw. Corticoide z.B. Betamethason sowie deren Ester (propionat, acetat, walerat), Hydrocortison, Methylprednisolon und deren Abkömmlinge, Triamcinolonacetamid, Benzalkoniumchlorid, Chlorhexidin, Chlorhexidin-Derivate, Dexpanthenol, Allantoin, Bisabolol, Triclosan; Antischuppenmittel, z. B. Climbazol oder Piroctone Olamine; Weiter geeignete Wirkstoffe sind Endocannabinoide (z.B. N-palmitoylethanol-amin), Mineralstoffe und Spurenelemente wie Kupfer, Zink, bzw. deren Derivate wie Zincidone^{®} (Zink PCA-Pyrrolidoncarbon-säure), Zinkglukonat oder Kupfergluconat; adstringierende und sebumregulierende Substanzen wie Acnacidol^{®} 101 (Propylene Glycol, Hydroxydecanoic Acid), Asebiol^{®} BT (Hydrolyzed Yeast Protein, Pyridoxine, Niacinamide, Glycerin, Panthenol, Propylene Glycol, Allatoin, Biotin), Lipacide^{®} C8C0 (Caproyl Collagen Aminoacids), Sebosoft^{®} (Glycerin, Aqua, Macrogol-8, Caprylyl Glycol, Sebacic Acid, Sodium Polyacrylate), Sepi Control A5 (Capryloylglycine, Methylglycine, Cinnamonum zeylanicum); kühlende/ beruhigende Wirkstoffe wie Frescolat^{®} ML (Menthyl Lactate) oder Eashave^{®} (Sodium Hyaluronate, Wheat Germ Extrakt, Saccharomyces Cerevisiae Extrakt); Durchblutungsfördernde Stoffe, z. B. Nikotinsäurederivate wie Methyl- oder Tocopherylnikotinat, Alpha- und Betahydroxysäuren und deren Derivate, z. B. Glykol-, Äpfel-, Zitronen-, Wein-, Milchsäure, Salicylsäure, Isopropylbenzylsalicylate, C12-13 Alkyl Lactate (Cosmacol^{®} ELI) oder auch antiphlogistische und antibakterielle Substanzen wie Triterpene, z. B. Ursolsäure, Glycyrrhizinsäure oder Glycyrrhetinsäure und deren Derivate, z. B. Stearyl Glycyrrhetinate, Kaliumglycyrrhinat; Pantothensäurederivate, z. B. D-Panthenol, Panthenyltriacetat; Allatoin; Bisabolol; Azulene, z. B. Chamazulen oder Guajazulen; Phytosphingosine;
   Weitere Wirkstoffe sind antioxidativ sowie zellschützend wirkende Stoffe wie Polyphenole, Flavonoide, z. B. Rutin, Ferulasäure und deren Ester oder Isoflavone wie Soja-Isoflavone oder Coenzym Q 10 ;
   Lipidtransferproteine können ebenfalls als wirksame Zusätze eingesetzt und insbesondere ausgewählt werden aus tierischen, pflanzlichen und mikrobiellen Proteinen. Diese können ggf. auch synthetisch oder auch gentechnologisch gewonnen werden. Dazu gehören insbesondere Lipidtransferprotein(e) aus Getreide, Knollenfrüchten oder Früchten, Proteinen aus Milch, Proteinen aus Seide, Mikroorganismen, marinen Quellen oder Mischungen hiervon. Ganz besonders geeignet sind hier Milchproteine und Getreide-, insbesondere Hafer- oder Gersteproteine der genannten Art oder Mischungen hiervon. Derartige Produkte sind charakterisiert und beschrieben in der DE 103 24 256 A1, welche hier inkorporiert ist. In einer besonders bevorzugten Ausführungsform werden ein oder mehrere Lipidtransferproteine wie beschrieben, insbesondere die bevorzugten, mit einem oder mehreren Lecithinen, Sphinglipiden, Cholesterinen, Phospholipiden, Sphingolipiden, Gangliosiden, Cerebrosiden, Ceramiden lipophilen oder amphiphilen Pflanzenstoffen kombiniert eingesetzt. Dabei können ggf. noch Zusatzstoffe wie Konsistenzregler, Konservierungsmittel, hautschützende Substanzen, Farb-Parfümstoffe eingesetzt werden. Damit können insbesondere kosmetische Zubereitungen in Form von Cremes, Lotionen für den Einsatz auf der Haut zur Pflege, insbesondere bei Altershaut, gestresster Haut erhalten werden.
   In einer weiteren Ausführungsform können als Wirkstoff Huminsäureprodukte, z. B. erhalten als Moorpräparate durch Zusatz von Torfextrakt, oder aus anderen Quellen wie fossilen Braunkohlematerialien, eingesetzt werden. Diese eignen sich insbesondere zur Herstellung von Emulsionszubereitungen für Bad/ Dusche wie Badezusatz / Duschzusatz.

### Herstellung

Die beschriebenen Grundlagen werden auf klassische Weise hergestellt, indem man in eine Wasserphase, welcher ggf. temperaturstabile gewünschte wasserlösliche Zusätze (Zusatzstoffe/ Wirkstoffe) beigefügt werden, mit einer oder mehreren Emulsionsionsstabilisator - Zuckerverbindungen bei Raumtemperatur, üblicherweise unter Erwärmen auf 55 bis 70 ° C, und Rühren hinzufügt und sodann eine Fettphase wie beschrieben, ggf. unter Zusatz dort löslicher temperaturstabiler Zusätze (Zusatzstoffe/ Wirkstoffe), ebenfalls bei 55-70°C und unter Rühren inkorporiert. Unter weiterem Rühren wird das emulgierte System auf Raumtemperatur abgekühlt. In Abhängigkeit von der Zusammensetzung des Präparates kann eine Homogenisierung mit Hilfe eines Intensivrührers wie z.B. Ultra-Turrax vorgenommen werden. Sofern eine pflegende, kosmetische oder dermatologische Anwendung gewünscht ist, können hierbei gewünschte wasser- und/oder fettlösliche Wirkstoffe/Zusätze (Zusatzstoffe) inkorporiert werden, so dass man direkt die gewünschte hydrophile oder ambiphile Emulsion erhält.

### Anwendung

Die erfindungsgemäße Grundlage kann als pflegende, kosmetische oder dermatologische Creme, Lotion oder flüssige Emulsion, balneologischer Zusatz (Badezusatz, Duschzusatz) mit anwendungsbezogener Viskosität, bei Säugern, insbesondere bei Menschen, eingesetzt werden. Die Anwendung kann je nach Wassergehalt und ggf. hinzugesetzten Wirkstoffen abgestimmt werden. Die Applikation erfolgt entweder direkt durch Auftragen auf die Haut /Schleimhaut oder nach Verdünnung, z.B. mit Wasser als Badezusatz oder für Duschzwecke. Aufgrund der speziellen Kombination an Lipiden und Emulsionsstabilisatoren werden im Wesentlichen nicht irritierende, hypoallergene Substanzen verwendet, welche auch im Lebensmittelbereich Verwendung finden. Die neue natürliche Grundlage weist daher eine Verträglichkeit auf, welche bei einer oralen Anwendung gefordert und insofern topisch bisher noch nicht erreicht worden ist. Überraschenderweise können trotz des (vorzugsweisen) Verzichts auf bisher als wesentlich genannte Verbindungen wie Paraffine, Vaseline, Cetyl/ Stearylalkohole, Glycerolpartialester konsistente, homogene und stabile Produkte erhalten werden, welche darüber hinaus überraschender Weise anisotrope Strukturen in Form von Nanosomen (Nanobionen), Mikrosomen (Mikrobionen) auszubilden vermögen. Mit diesen ist ein besonders effektiver Stofftransport möglich. So können die erfindungsgemäßen Produkte eingesetzt werden zur kosmetischen Pflege bzw. unterstützenden Anwendung oder auch zur Behandlung von normaler, trockener, fettiger, irritierter Haut, zur Verbesserung des Erscheinungsbildes und Zustands gestresster oder alternder Haut / Haare, bei der Dusche, als Badezusatz, als Sonnenschutzmittel, zur unterstützenden Anwendung bei Akne, Psoriasis, Neurodermitis, Allergien, bakteriell oder durch andere Mikroorganismen verursachten Störungen der Haut, Schleimhaut/Haare oder auch dermatologisch zur Behandlung von Erkrankungen der Haut, Schleimhäuten und/oder Haare, wie z.B. Akne, Psoriasis, Neurodermitis, altersbedingten Krankheiten, Allergien, bakteriellen oder anderen durch Mikroorganismen verursachten Krankheiten, die mit Hilfe von. z.B. Antibiotika, Antimykotika, Antiseptika und / oder Antiallergika behandelt werden, ohne dass anwendungsbezogene Änderungen in der Grundlage erforderlich werden.

### Beispiele

Die Erfindung wird anhand der nachfolgenden Beispiele (ohne Beschränkung hierauf) näher erläutert. Dabei wurden die einzelnen Zubereitungen wie oben beschrieben hergestellt. Dabei bedeuten:
- Crodesta F110 INCI:: Sucrose stearate
- Glucate SS: Methyl Glucose Sesquiisostearate
- Glucate DO:: Methyl Glucose Dioleate
- HEC:: Hydroxyethylcellulose
- Kokosnussöl: Cocos nucifera
- Sojaöl: Glycine soja
- Jojobaöl:: Buxus Chinensis oder Simmondsia Chinensis
- Sesamöl: Sesamum indicum
- Shea-Butter:: Butyrospermum Parkii
- Tylose 4000: Hydroxyethylcellulose
- Plantacare®2000: Decylglucoside

### Beispiel 1

Folgende Creme wurde erhalten aus:

| | |
|---|---|
| Crodesta F110 | 8,0 |
| Glucate SS | 1,0 |
| Kokosnussöl | 4,0 |
| Sesamöl | 4,0 |
| Shea butter | 2,5 |
| Glycerol | 5,0 |
| HEC | 0,2 |
| Ethanol | 15,0 |
| Cholesterol | q.s |
| Wasser | 60,3 |

### Beispiel 2

Eine Creme wurde erhalten aus:

| | |
|---|---|
| Crodesta F110 | 6 |
| Glucate SS | 3,0 |
| Kokosnussöl | 6,0 |
| Jojobaöl | 6,0 |
| Glycerol | 10 |
| HEC | 0,2 |
| Ethanol | 15,0 |
| α-Tocopherol | 0,03 |
| Wasser | 53,77 |

### Beispiel 3

Eine Creme wurde erhalten aus:

| | |
|---|---|
| Crodesta F110 | 6 |
| Glucate SS | 3,0 |
| Kokosnussöl | 6,0 |
| Sesamöl | 6,0 |
| Sheabutter | 6,0 |
| Glycerol | 10 |
| HEC | 0,2 |
| Wasser | 62,8 |

### Beispiel 4

Eine Lotion wurde erhalten aus:

| | |
|---|---|
| Crodesta F110 | 6 |
| Glucate SS | 3,0 |
| Kokosnussöl | 6,0 |
| Sesamöl | 6,0 |
| Glycerol | 10 |
| HEC | 0,2 |
| Wasser | 68,8 |

### Beispiel 5

Eine Creme wurde erhalten aus:

| | |
|---|---|
| Crodesta F110 | 7 |
| Glucate DO | 4,0 |
| Kokosnussöl | 12,5 |
| Jojobaöl | 12,5 |
| Glycerol | 10 |
| HEC | 0,2 |
| Wasser | 53,8 |

### Beispiel 6

Eine Lotion wurde erhalten aus:

| | |
|---|---|
| Crodesta F110 | 7 |
| Glucate SS | 4,0 |
| Kokosnussöl | 6,0 |
| Jojobaöl | 6,0 |
| Glycerol | 10 |
| Bienenwachs | 5 |
| HEC | 0,2 |
| Wasser | 61,8 |

### Beispiel 7

Eine Creme wurde erhalten aus:

| | |
|---|---|
| Crodesta F110 | 6 |
| Glucate DO | 3,0 |
| Kokosnussöl | 7,0 |
| Sojaöl | 7,0 |
| Glycerol | 5 |
| HEC | 0,2 |
| Ethanol | 15 |
| Wasser | 56,8 |

### Beispiel 8

Eine Lotion wurde erhalten aus:

| | |
|---|---|
| Crodesta F110 | 6 |
| Glucate SS | 3,0 |
| Sesamöl | 7,0 |
| Kokosöl | 7,0 |
| Glycerol | 5 |
| HEC | 0,2 |
| α- Tocopherol | 0,03 |
| Wasser | 71,77 |

Für die folgenden Beispiele 9 bis 11 sind die anisotropen Strukturen (Vesikel), nachweisbar mittels Polarisationsmikroskop, anhand der hellen Punkte in Fig. 1 (Beispiel 9), Fig.2 (Beispiel 10) und Fig. 3 (Beispiel 11) dargestellt.

### Beispiel 9 (Creme)

| | |
|---|---|
| Crodesta F110 | 7 |
| Glucate SS | 4,0 |
| Kokosnussöl | 6,0 |
| Jojobaöl | 6,0 |
| Glycerol | 10 |
| Tylose 4000 | 0,2 |
| Phenylethylalkohol | 0,4 |
| Wasser | 66,4 |

### Beispiel 10 (Lotion)

| | |
|---|---|
| Crodesta F110 | 6 |
| Glucate SS | 3,0 |
| Kokosnussöl | 6,0 |
| Sesamöl | 6,0 |
| Biennenwachs | 5,0 |
| Glycerol | 10 |
| Tylose 4000 | 0,2 |
| Wasser | 63,8 |

### Beispiel 11 (Creme)

| | |
|---|---|
| Crodesta F110 | 6 |
| Glucate SS | 3,0 |
| Kokosnussöl | 6,0 |
| Sesamöl | 6,0 |
| Kakaobutter | 3,0 |
| Glycerol | 10 |
| Tylose 4000 | 0,2 |
| Wasser | 65,8 |

### Beispiel 12

Eine Lotion wurde erhalten aus:

| | |
|---|---|
| Plantacare®2000 | 6 |
| Glucate SS | 3,0 |
| Kokosnussöl | 6,0 |
| Jojobaöl | 6,0 |
| Glycerol | 10 |
| HEC | 0,2 |
| α-Tocopherol | 0,03 |
| Wasser | 68,77 |

## Patentansprüche

1. Hydrophile bzw. ambiphile Grundlage auf natürlicher Basis, umfassend:
a) 1 bis 50 % eines oder mehrerer nativer Lipide (Fettphase);
b) 0,1 bis 30 % eines oder mehrerer Emulsionsstabilisatoren, ausgewählt aus
bi) Zuckerestern, substituierten Zuckerestern von C₁₂-C₁₈ gesättigten, ungesättigten, partial gesättigten Fettsäuren oder Mischungen hiervon;
bii) Zuckerethern von C₈-C₂₀ gesättigten, ungesättigten, partial gesättigten Fettalkoholen oder Mischungen hiervon;
oder Mischungen aus bi), bii);
c) 10 bis 95 Gew.% Wasser;
d) 0 bis 55 Gew.% Zusatzstoffe.

2. Hydrophile Grundlage gemäß Anspruch 1, **dadurch gekennzeichnet, dass** sie 5 bis 40 Gew.%, eines oder mehrerer Lipide; und 1 bis 25 Gew. % eines oder mehrerer Emulsionsstabilisator(en) aufweist.

3. Hydrophile Grundlage gemäß Anspruch 1 oder 2, **dadurch gekennzeichnet, dass** sie 2 bis 20 Gew.% eines oder mehrerer Emulsionsstabilisatoren und 1 bis 20 Gew.% Zusatzstoffe aufweist.

4. Hydrophile Grundlage gemäß einem der Ansprüche 1 bis 3, **dadurch gekennzeichnet, dass** das oder die Lipide ausgewählt sind aus natürlichen Ölen wie Mandelöl, Sojaöl, Kokosnussöl, Jojobaöl, Avocadoöl, Erdnussöl, Traubenkernöl, Sonnenblumenöl, Borretschöl, Sesamöl, Olivenöl, Aprikosenkernöl, Maiskeimöl, Weizenkeimöl, Walnussöl, Palmöl, Macadamianussöl, Palmkernöl, Haselnussöl, Hagebuttenkernöl, Baumwollsamenöl.

5. Hydrophile Grundlage gemäß einem der Ansprüche 1 bis 4, **dadurch gekennzeichnet, dass** ein oder mehrere Emulsionsstabilisatoren, ausgewählt aus Glucose-, Methylglucose- oder Saccharose- C₁₂-C₁₈-Säureestern; C₈C₂₀-Glucoserethern oder Mischungen hiervon, enthalten sind.

6. Kosmetische oder dermatologische Zubereitungen, enthaltend: 45 bis 100 Gew. % einer hydrophilen bzw. ambiphilen Grundlage a)+b)+c) gemäß einem der Ansprüche 1 bis 5, sowie 0 bis 55 Gew. % Zusatzstoffe d).

7. Zubereitungen gemäß Anspruch 6, **dadurch gekennzeichnet, dass** 45 bis 99 Gew.% einer Hydrophilen bzw. ambiphilen Grundlage a)+b)+c) gemäß einem der Ansprüche 1 bis 5 und 1 bis 55 Gew. % Zusatzstoffe enthalten sind.

8. Zubereitungen gemäß Anspruch 6 oder 7, **dadurch gekennzeichnet, dass** die Zusatzstoffe ausgewählt sind aus einem oder mehreren Wirkstoffen, insbesondere einem oder mehreren pflegenden, kosmetischen und/ oder therapeutischen Wirkstoffen, ferner auch aus der Gruppe umfassend Konsistenzregler, Konservierungsstoffe, Farbstoffe, Parfümstoffe Schaumregler oder Mischungen hiervon oder Mischungen hiervon und mit einem oder mehreren Wirkstoffen.

9. Zubereitungen gemäß Anspruch 8, **dadurch gekennzeichnet, dass** der oder die Wirkstoffe ausgewählt sind aus ätherischen Ölen, Pflanzenextrakten und/oder fossilen Stoffen wie Torfprodukte, UV Filtern, Antibiotika, Antimykotika, Antiseptika, Virostatika, haarwuchsfördernden Substanzen, entzündungshemmenden Stoffen, Hormonen, Antischuppenmitteln, zellschützenden und antioxidativen Substanzen, Pflegestoffen, Mineralien, Vitaminen oder Mischungen hiervon, Feuchthaltern, Rückfettern, ein oder mehrere Lipidtransferproteinen, einem oder mehreren Lecithinen, oder Kombinationen hiervon.

10. Zubereitungen nach einem der Ansprüche 6 bis 9, **dadurch gekennzeichnet, dass** ein oder mehrere Lipidtransferproteine, ausgewählt aus pflanzlichen Proteinen, Milchproteinen und Mischungen hiervon und ein oder mehreren Lecithine oder Lecithinanaloga, ausgewählt aus Lecithinen, Cholesterinen, Phospholipiden, Sphingolipiden, Gangliosiden, Cerebrosiden, Ceramiden, lipophilen oder amphiphilen Pflanzenstoffen enthalten sind.

11. Zubereitungen nach Anspruch 10, **dadurch gekennzeichnet, dass** weiterhin ein oder mehrere fettlösliche und/oder wasserlösliche Vitamine enthalten sind.

12. Zubereitungen nach einem der Ansprüche 6 bis 9, **dadurch gekennzeichnet, dass** als Wirkstoff Torfprodukte oder analoge Huminsäurequellen enthalten sind.

13. Verfahren zur Herstellung von Grundlagen und Zubereitungen gemäß einem der Ansprüche 1 bis 12, **dadurch gekennzeichnet, dass** man einer Wasserphase, welcher ggf. wasserlösliche Zusätze (Zusatzstoffe/ Wirkstoffe) beigefügt werden, eine oder mehrere Emulsionsionsstabilisator - Zuckerverbindungen bei Raumtemperatur, ggf. unter Erwärmen auf 55 bis 70 ° C, und Rühren hinzufügt, und sodann eine Fettphase wie beschrieben, ggf. unter Zusatz dort löslicher Zusätze (Zusatzstoffe/ Wirkstoffe), bei Raumtemperatur, ggf. bei 55-70°C unter Rühren inkorporiert und nach dem Abkühlen und ggf. Homogenisieren ggf. eventuell temperaturlabile wasser- und/oder fettlösliche Zusätze (Wirkstoffe/Zusatzstoffe) inkorporiert werden.

14. Verwendung von Grundlagen oder Zubereitungen gemäß einem der Ansprüche 1 bis 12 zur pflegenden, kosmetischen oder balneologischen Anwendung auf Haut, Haare, insbesondere des Menschen.

15. Verwendung gemäß Anspruch 14, zur pflegenden, kosmetischen Pflege / unterstützenden Anwendung von / bei normaler, trockener, fettiger und/oder irritierter Haut, zur Verbesserung des Erscheinungsbildes und Zustands gestresster, alternder Haut, als Sonnenschutzmittel oder Bade- bzw. Duschzusatz.

16. Verwendung gemäß Anspruch 15 zur pflegenden/ kosmetischen unterstützenden Anwendung bei Akne, Psoriasis, Neurodermitis, Allergien, bakteriellen oder durch andere Mikroorganismen verursachten Störungen der Haut des Menschen.

17. Verwendung von Grundlagen oder Zubereitungen gemäß einem der Ansprüche 1 bis 12 zur Herstellung eines Mittels zur dermatologisch ― pharmazeutischen oder balneologischen Behandlung von Erkrankungen der Haut, Schleimhäuten und/oder Haare, wie z.B. Akne, Psoriasis, Neurodermitis, bakteriellen oder anderen durch Mikroorganismen verursachten z. B. mit Antibiotika, Antimykotika, Antiseptika und/oder Antiallergika behandelten Krankheiten.
